(19) Europäisches Patentamt
European Patent Office
Office européen des brevets

(11) **EP 3 904 878 B1**

(12) **EUROPEAN PATENT SPECIFICATION**

(45) Date of publication and mention
of the grant of the patent:
**21.02.2024  Bulletin 2024/08**

(21) Application number: **19902777.2**

(22) Date of filing: **10.07.2019**

(51) International Patent Classification (IPC):
**G01N 33/543** *(2006.01)*    **G01N 33/76** *(2006.01)*

(52) Cooperative Patent Classification (CPC):
**G01N 33/54386; G01N 33/76**

(86) International application number:
**PCT/KR2019/008465**

(87) International publication number:
**WO 2020/138614 (02.07.2020 Gazette 2020/27)**

(54) **IMMUNOCHROMATOGRAPHY STRIP FOR PREGNANCY DIAGNOSIS WITH MULTIPLE TEST LINES, AND PREGNANCY DIAGNOSIS KIT COMPRISING SAME**

IMMUNCHROMATOGRAFISCHER STREIFEN ZUR SCHWANGERSCHAFTSDIAGNOSE MIT MEHRFACHEN TESTLINIEN UND SCHWANGERSCHAFTSDIAGNOSEKIT DAMIT

BANDELLETTE D'IMMUNOCHROMATOGRAPHIE POUR DIAGNOSTIC DE GROSSESSE, COMPORTANT DE MULTIPLES TRAITS DE TEST, ET KIT DE DIAGNOSTIC DE GROSSESSE LA COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2018  KR 20180172576**

(43) Date of publication of application:
**03.11.2021  Bulletin 2021/44**

(73) Proprietor: **Protia Inc.**
**Seoul 07528 (KR)**

(72) Inventors:
• **LIM, Kook Jin**
  **Seoul 06291 (KR)**
• **KIM, Tae Hyong**
  **Seoul 04179 (KR)**
• **PARK, Dong Suk**
  **Seongnam-si, Gyeonggi-do 13586 (KR)**
• **CHOI, Dong Seob**
  **Siheung-si, Gyeonggi-do 14998 (KR)**
• **KIM, Mi Jeoung**
  **Seoul 02638 (KR)**
• **PARK, Young Woo**
  **Seoul 07402 (KR)**

(74) Representative: **J A Kemp LLP**
**80 Turnmill Street**
**London EC1M 5QU (GB)**

(56) References cited:
**CN-A- 102 890 157      CN-A- 102 890 157
CN-A- 103 777 002      CN-A- 106 053 794
CN-A- 108 931 654      KR-A- 20020 014 004
KR-A- 20160 108 695    KR-A- 20160 108 695
US-A1- 2007 172 963**

• **STENMAN ULF-HÅKAN ET AL: "Determination of human chorionic gonadotropin", BEST PRACTICE & RESEARCH CLINICAL ENDOCRINOLOGY & METABOLISM, BAILLIERE TINDALL, LONDON, GB, vol. 27, no. 6, 26 October 2013 (2013-10-26), pages 783-793, XP028784595, ISSN: 1521-690X, DOI: 10.1016/J.BEEM.2013.10.005**
• **NERENZ, R. D.: "Characterizing urinary hCGbeta cf patterns during pregnancy", Clinical Biochemistry, 2016, pages 777-781, XP029617201, DOI: 10.1016/j.clinbiochem.2016.04.006**

**Description**

[Technical Field]

The present invention relates to a pregnancy diagnostic kit for diagnosing pregnancy more accurately by measuring human chorionic gonadotropin (hCG) as well as hCG β core fragment (hCGβcf)together in pregnancy diagnosis.

[Background Art]

The immunochromatographic assay is a method capable of qualitatively and quantitatively analyzing a small amount of an analyte in a short period of time using an antigen-antibody reaction. Pregnancy diagnostic kits, which are widely used in homes and hospitals, use such an immunochromatographic assay. In the case of immunochromatography strips used in general pregnancy diagnostic kits, human chorionic gonadotropin (hCG), the amount of which increases in urine and serum during pregnancy, is used as a target substance to be detected. The antibody binding to hCG is immobilized on a membrane pad in a line form (test line), and another antibody is bound to a labeling material such as gold particles and the like and immobilized on a conjugate pad, and then the sample is developed. The developed sample passes through a conjugate pad and is developed by forming a complex of hCG-anti hCG antibody-gold particle in the sample, and the complex is captured by an anti-hCG antibody immobilized on the test line to form a 'test line-immobilized antibody-anti hCG antibody-gold particle.' This is called the "sandwich reaction". When hCG is present at a concentration that can be determined as pregnancy in the sample, the test line appears red by gold particles, and it is determined as pregnant. In addition, the control line always captures the gold particle antibody regardless of the concentration of hCG in the sample, and since the red color appears by the gold particle, the normal termination of the test can be confirmed. The analysis strip showing the result can be detected by the naked eye or using a sensor.

Human chorionic gonadotropin (hCG) is a 36.5 kD glycoprotein, composed of two subunits, alpha (α) and beta (β), and it functions similarly to luteinizing hormone (LH), such as luteinizing of the ovary, inducing ovulation, promoting androgen secretion in interstitial cells of the testis, and the like. The hCG is produced and secreted from the placental trophoblast cells when a woman becomes pregnant and is present in the mother's blood, urine, and amniotic fluid. Other than the above, it is known to increase in trophoblastic diseases such as hydatidiform mole and choriocarcinoma and in various embryonic cell tumor patients, and it is also present in a very small amount in the blood and urine of normal men and non-pregnant women. The hCG is produced in the placenta and released through the blood into urine, and while passing through this step, various decomposition products and variants are generated, and there is a difference in the hCG forms shown in the placenta, blood, and urine. The α subunit has two N-nicked oligosaccharide side chains, and the β subunit distinguishes hCG from other glycol proteins in a unique form with 145 amino acid linkages on six legs. Depending on the change of this amino chain, the placenta exhibits the forms of large free α and non-nicked free β, and the forms of regular free α and nicked free β are added in the blood, and in particular, it is present in the form of hCG β core fragment (hCGβcf) in urine (Journal of the Korean Society of Emergency Medicine: 2011, Vol. 22, No. 5, 503-507).

In urine before 5 weeks of pregnancy, there are relatively few hCG β core fragments (hCGβcf), but in urine after 6 weeks of pregnancy, hCGβcf is present at a higher amount than normal hCG (intact hCG; I-hCG), and especially after 8 weeks, it is present at an amount 3 times or more than normal hCG. This high concentration of hCGβcf may interfere with the measurement of normal hCG and cause false negative errors in pregnancy diagnosis (J. Emergency Medicine 2013, 44:155).

Accordingly, as a result of continuously researching a method of manufacturing a pregnancy diagnostic kit capable of accurately determining whether to be pregnant without being affected by hCGβcf, which is present at a high concentration in urine, the present inventors completed the present invention by confirming that when the chromatography strip for diagnosing pregnancy having multiple test lines according to the present invention was used, it was possible to accurately determine whether to be pregnant even when hCGβcf was present at a high concentration.

US 2007/172963 relates to a lateral flow assay for detecting the presence of an analyte in a liquid test sample. The lateral flow assay detects the presence or absence of a target analyte in a liquid sample, in part, by encompassing a reference region of immobilized, non-diffusible analyte that allows for detection of any factors that interfere with the interaction and binding of the analyte to the labeled capture reagent. Any influences on the interaction and binding of the analyte that is free in solution in the liquid test sample to its complementary labeled reagent will be encountered in parallel in the binding between the immobilized analyte in reference region to the labeled reagent as it diffuses through the reference region. In one embodiment, the lateral flow assay of the invention is a urine-based human Chorionic Gonadotropin (hCG) assay.

KR 2016/0108695 relates to a different inspection line-included immunochromatographic strip, a pregnancy diagnosis kit comprising the immunochromatographic strip, and a method for performing a qualitative or quantitative analysis on human chorionic gonadotropin in a testing object using the same. The immunochromatographic strip for pregnancy diagnosis including different inspection lines.

CN 106053794 relates to a reagent card for accurately detecting a test object, a kit and application. A sample pad, an antibody carrier film and water absorption paper are successively adhered to a PVC bottom plate from one end to the other end, wherein the sample pad is a sample loading area, and the sample pad is sprayed with a test object antibody-1 coupling marker and a biotin coupling marker; the antibody carrier film is provided with three lines which are successively a line T1, a line T2 and a line C from the sample pad to the water absorption paper, or are the line T1, the line C and the line T2; the line T1 is a detection line, and a test object antibody 2 is smeared on the position of the line T1; an antigen of the test object is smeared on the position of the line T2; streptavidin is smeared on the position of the line C.

CN 108931654 relates to a production method of an early pregnancy detection kit using urine as a detection sample. The production method adopts the principle of a colloidal gold immunochromatography technology and uses a double-antibody sandwich method technology, a T1 detection line of a nitrocellulose membrane is coated with an anti-beta-HCG core fragment monoclonal antibody, a T2 detection line of the nitrocellulose membrane is coated with an anti-alpha-HCG monoclonal antibody, and a quality control line of the nitrocellulose membrane is coated with a goat anti-mouse IgG polyclonal antibody. The document further relates to the product obtained through the production method, and a use of the product.

CN 102890157 relates to a test strip and a method for fast quantitative detection of human chorionic gonadotropin (HCG). The test strip comprises a sample loading pad, a marker pad, an NC membrane, a sample suction pad and a plastic plate. The sample loading pad, the marker pad, the NC membrane and the sample suction pad are orderly adhered to the plastic plate. The marker pad is coated with colloidal gold-marked mouse anti-human HCG-beta monoclonal antibodies. The NC membrane is coated with a detection line T composed of mouse anti-human HCG-alpha antibodies and a quality control line C composed of goat anti-mouse IgG antibodies. On the NC membrane, a detection line T side close to the marker pad is provided with a sample loading indication line for indicating a sample loading position.

[Disclosure]

[Technical Problem]

Accordingly, an object of the present invention is to provide a chromatography strip for diagnosing pregnancy with high accuracy by excluding interference of hCGβcf present at a high concentration in urine.

Another object of the present invention is to provide a pregnancy diagnostic kit including the chromatography strip.

Still another object of the present invention is to provide a method for diagnosing pregnancy using the chromatography strip or pregnancy diagnostic kit.

[Technical Solution]

In one aspect for solving the above problems, the present invention provides a chromatography strip for diagnosing pregnancy including a conjugate pad and a detection pad.

In the chromatography strip for diagnosing pregnancy of the present invention, the conjugate pad includes a first anti-human chorionic gonadotropin (hCG) monoclonal antibody and a signal detection labeling material, the detection pad is provided with test line 1 and test line 2 that are isolated, the test line 1 includes a second anti-hCG monoclonal antibody, and the test line 2 includes human chorionic gonadotropin (hCG).

The "first anti-hCG monoclonal antibody" may be used interchangeably with the "first anti-hCG antibody" and refers to an antibody capable of recognizing the β-core fragment (hCGβcf) region of human chorionic gonadotropin. Therefore, the first anti-hCG monoclonal antibody of the present invention may specifically bind to hCGβcf as well as normal hCG.

The "second anti-hCG monoclonal antibody" may be used interchangeably with the "second anti-hCG antibody", and refers to an antibody capable of specifically binding by recognizing another region of hCG other than the hCGβcf region. Therefore, the second anti-hCG monoclonal antibody of the present inventionspecifically binds to normal hCG only and does not bind to hCGβcf. In one preferred exemplary embodiment of the present invention, the other region of the hCG may be a part of the normal hCG β subunit.

In the immunochromatography strip for diagnosing pregnancy of the present invention, the first anti-hCG monoclonal antibody is linked to a signal detection labeling material before or during the development of a sample to form a first conjugate, and a complex in which hCG or hCGβcf is bound to the first conjugate is formed when hCG or hCGβcf is present in the sample. In addition, as the concentration of hCG or hCGβcf increases in the sample, the number of the formed complexes increases, and conversely, the number of bare conjugates which are not bound to hCG in the sample decreases.

A complex in which normal hCG is bound to the first conjugate is captured on test line 1 to indicate a signal, and a complex in which hCGβcf is bound to the first conjugate is not captured on test line 1. In addition, a bare conjugate which is not bound to hCG or hCGβcf in the sample reacts with hCG immobilized at a certain amount on test line 2, thereby being captured on test line 2 to indicate a signal.

As used herein, the term "conjugate" refers to a conjugate formed by linking the labeling material and the ligand to each other, and the conjugate includes a first conjugate in which a labeling material for signal detection and a first anti-hCG antibody are linked to each other. The labeling material and the first anti-hCG antibody may be physically or chemically linked. That is, the labeling material and the first anti-hCG antibody may be linked by passive adsorption, and the labeling material may be modified to have a reactive group to be covalently bonded to the first anti-hCG antibody. However, it is not limited thereto, and the linkage of the labeling material and the first anti-hCG antibody may be performed using a method known to those skilled in the art. However, the labeling material and the first anti-hCG antibody may be present in the conjugate pad by being linked in the form of a conjugate before developing the sample on the chromatography strip for diagnosing pregnancy. Alternatively, they may be present in the conjugate pad in a separated state without being linked to each other, and as the sample is developed, they may be linked to each other to form a conjugate, or may exist in a solution phase and be added to the conjugate pad before use. In either case, during sample development, the labeling material and the first anti-hCG antibody may move to the detection pad in the state of a conjugate, and it is because they are not immobilized in the conjugate pad.

As used herein, the term "labeling material" refers to a material that generates a signal that may be detected by the naked eye or using a sensor. As the labeling material, gold colloid (gold particles), latex particles, colored polystyrene microparticles, enzymes, fluorescent dyes, conductive polymers, luminescent materials, or magnetic particles may be used, but it is not limited thereto. In addition, the signal may be generated by itself due to the inherent characteristics of the labeling material such as light emission and the like, or may be generated by an external stimulus such as fluorescence and the like.

In the chromatography strip for diagnosing pregnancy of the present invention, the conjugate pad may further include a material that may be used as an internal control and a signal detection labeling material. The internal control material and the labeling material are linked to each other before or during sample development to form a second conjugate, and the second conjugate is captured by a ligand of the control line, thereby confirming the development of the sample.

As used herein, the term "control line" refers to a part that emits a constant signal regardless of the concentration of the sample or hCG in the sample. The control line may be formed using a method similar to the test line and the heterogeneous test line. However, while not binding to hCG, the control line may be formed by immobilizing a material that may be captured by specifically or nonspecifically binding to a separate material bound to gold particles, as a second conjugate that moves along the detection pad by a mobile phase together with the sample. The material immobilized on the control line may be referred to as a ligand. As a result, it is formed by immobilizing a ligand capable of emitting a constant signal regardless of the presence or absence of chorionic gonadotropin in the sample. As the control line, anti-rabbit IgG, anti-chicken IgY, streptavidin, bovine serum albumin and the like may be used. The control line may be formed in two or more by varying the concentration of the ligand to be immobilized. The higher the concentration of the ligand immobilized on the control line, the more the control line may constantly emit a strong signal.

In the chromatography strip for diagnosing pregnancy of the present invention, the position of the test line and the position of the control line may be appropriately selected depending on the antigen-antibody reaction used and the like, but is not limited thereto.

Unlike the pregnancy diagnostic kit using a conventional analytical strip having a detection pad formed only with a control line that can confirm the development of a test line and a sample, the present invention is characterized in that by having two test lines consisting of test line 1 that detects only normal hCG and test line 2 that simultaneously detects hCGβcf, it may perform the determination of pregnancy more reliably.

In one exemplary embodiment of the present invention, when the first conjugate present in the conjugate pad moves to a liquid sample (such as urine of a woman of childbearing age, *etc.*) and a detection pad, it binds to the β-core fragment site of hCG in the sample to form a hCG-first anti-hCG antibody-gold particle complex. The complex is captured by a second anti-hCG antibody immobilized on test line 1 while being developed (sandwich immune reaction), and the first anti-hCG antibody-gold particle conjugate that is not bound to hCG in the sample is captured on test line 2 while being developed (competitive immune reaction). Since the amount of the first anti-hCG antibody-gold particle conjugate present in the conjugate pad is determined and used in a specific amount, as the concentration of hCG in the sample increases, it may show an inversely proportional relationship where the number of complexes, which are formed by the first anti-hCG antibody-gold particle conjugate present in the conjugate pad binding to hCG in the sample, increases, and conversely, the number of bare conjugates which are not bound to chorionic gonadotropin decreases. On the test line 1, the normal hCG β subunit may be measured by the sandwich immune method, and on the test line 2, the hCG and a derivative thereof may be simultaneously measured by the competitive immune method.

In one exemplary embodiment of the present invention, the signal intensity of the test line increases as the concentration of hCG in the sample increases, but when it increases above a certain concentration, it shows a phenomenon where the signal intensity rather decreases. The signal intensity from the test line 2, where the bare conjugate is captured, decreases as the concentration of hCG increases. In particular, it decreases under the influence of the hCGβcf concentration, which increases significantly after 6 weeks of pregnancy. As a result, it is possible to analyze hCG through signals from the labeling materials on the test line 1 and test line 2. More specifically, when the concentration of hCGβcf is low, it may not affect the sandwich immune test of test line 1, and thus, it is possible to accurately diagnose whether to be pregnant by measuring hCG with only test line 1. That is, when the test line 1 shows color development, it may be judged to be positive for pregnancy. However, when a large amount of hCGβcf is contained, it is possible to interfere with the immune test of the test line 1, and thus, the test line 1 and test line 2 may be combined to accurately diagnose pregnancy. That is, even when the test line 1 does not show color development, if the test line 2 is not visible or the intensity is below the control line, it is determined to be positive for pregnancy, and thus, it is possible to diagnose pregnancy more accurately.

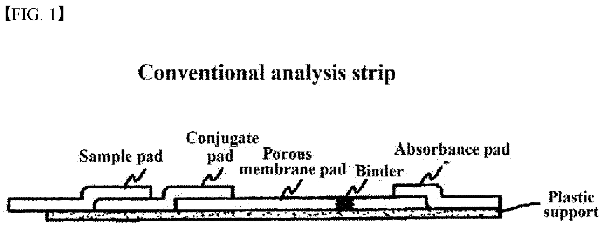

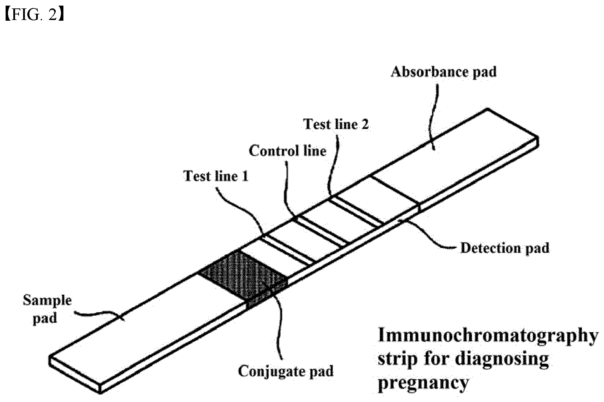

The constitution of the chromatography strip for diagnosing pregnancy of the present invention will be described in more detail. The chromatography strip for diagnosing pregnancy may include a sample pad into which a sample to be confirmed whether a target substance is included; a conjugate pad having one end connected to the sample pad; a detection pad having one end connected to the other end of the conjugate pad; an absorbance pad having one end connected to the other end of the detection pad and providing a driving force for transferring the sample from the sample pad; and a solid support located under the chromatography strip for diagnosing pregnancy. The constitutional diagrams for the chromatography strip for diagnosing pregnancy of the present invention are shown in [FIG. 1] and [FIG. 2].

The solid support may be formed of a material selected from the group consisting of nitrocellulose, nylon, PVDF, glass, and plastic. By manufacturing by attaching a strip on the solid support, the durability of the strip may be increased, and handling and storage may be facilitated. In addition, it is possible to facilitate the mounting of an additional outer case. As the plastic material that may be used as the solid support, a polypropylene film, a polyester film, a polycarbonate film, an acrylic film, and the like may be used, but is not limited thereto.

In another aspect, as a kit in which the chromatography strip for diagnosing pregnancy is additionally fixed in a case, the present invention provides a diagnostic kit in which a guide and a strip support are provided in a lower case, and a sample input port is provided in an upper case; and a result confirmation window is provided at a position corresponding to multiple test lines and control lines.

The chromatography strip for diagnosing pregnancy may additionally be fixed in a case. The inside of the lower case may be provided with multiple guides and/or strip supports for positioning and fixing or compressing the chromatography strip for diagnosing pregnancy in an appropriate position. Optionally, the guide and the strip support may be provided in the upper case at positions corresponding to the guide and strip support provided in the lower case. That is, the guide and/or strip support may be formed in the lower case or both the upper case and the lower case, if necessary. In addition, the upper case may be provided with a result confirmation window for detecting a signal from the labeling material at a position corresponding to the sample input port, the test line, and the control line. The sample input port is formed at one end of the detection pad, that is, on the other end of the absorbance pad based on the test line, and at the same time, it may be formed in the form of a hole, a slit, and the like at a position sufficiently spaced apart with the test line such that the sample may be developed along a membrane. The result confirmation window may also include multiple test lines and control lines on the detection pad, and may be formed to a size large enough to be discernable from the outside by the naked eye or a sensor. As long as multiple test lines and control lines may be identified, the size and shape thereof may be formed without limitation.

The upper and lower cases may be manufactured using a conventional plastic material, for example, polycarbonate, acrylonitrile butadiene styrene (ABS), and the like may be used, but are not limited thereto. The upper and lower cases may be separately manufactured and provided with engaging grooves, engaging projections, and the like, and combined by conventional means, and in some cases, these may be integrally manufactured.

As described above, chromatography uses the principle of chromatography in which a mobile phase including an analyte moves along a medium. Therefore, for analysis using a chromatography strip for diagnosing pregnancy, a mobile phase is required to move a sample including an analyte along the strip. Accordingly, the analysis kit of the present invention may further include a buffer solution. The buffer solution not only acts as a mobile phase for moving the sample along the chromatography strip for diagnosing pregnancy, but may also serve as a solvent for dissolving the conjugate, and may also serve as a diluent for diluting the sample if necessary. In addition, for example, when performing a whole blood analysis, a component for dissolving (lysis) blood cell components such as red blood cells and the like may be further included. As the buffer solution, a conventional buffer solution such as a phosphate buffered solution (PBS) having a concentration of 10 mM to 1 M, a nonionic or amphoteric surfactant, a mixture thereof, or the like may be used without limitation, and depending on the type of desired reactions such as antigen-antibody reactions and the like, the composition and usage ratio of the buffer solution may be appropriately selected.

In another aspect, as a method for diagnosing pregnancy using the chromatography strip for diagnosing pregnancy, the present invention provides a method for diagnosing pregnancy performed by including injecting a sample into the conjugate pad or a pad positioned before to develop; and confirming the presence or absence of a signal of a labeling material from multiple test lines and control lines.

In performing a specific analysis method, it may proceed in the following order. First, a liquid sample may be injected into the conjugate pad or a pad positioned before. That is, the liquid sample may be injected directly into the conjugate pad, and the sample may be introduced into the strip, but may preferably be injected before the conjugate pad, for example, in the sample pad to be introduced. In addition, the sample may be uniformly mixed by adding a buffer solution such as PBS, and introduced into the strip in the same manner.

As described above, when the sample is loaded (introduced) onto the chromatography strip for diagnosing pregnancy, development begins, and then it may be confirmed whether the development of the sample is performed well through a control line. If the sample is well developed, it may confirm the presence or absence of the signal of the labeling material from several test lines and control lines. If it corresponds to 1) below, it may be diagnosed as non-pregnant, and if it corresponds to 2) to 4) below, it may be diagnosed as pregnant.

1) If the control line and test line 2 appear, this indicates that hCG or hCGβcf is not included in the sample, and it may be diagnosed as non-pregnant.
2) If test line 1, test line 2, and the control line all appear, this indicates that hCG or hCGβcf is included in the sample, and it may be diagnosed as pregnant.
3) If test line 1 and the control line appear, this indicates that hCG is included in the sample, and it may be diagnosed as pregnant.
4) If only the control line appears, this indicates that hCG or hCGβcf is included at a high concentration in the sample, and it may be diagnosed as pregnant.

As a sample for analysis of the present invention, it may include not only a general aqueous solution, but also all biological samples such as whole blood, blood cells, serum, plasma, bone marrow, sweat, urine, tears, saliva, skin, mucous membranes, hair, and the like isolated from mammals, preferably humans, and preferably, it may be urine.

[Advantageous Effects]

In the present invention, a monoclonal antibody for detecting normal hCG and hCGβcf, which are present in the urine of a pregnant woman, are constituted to be paired with each other, and by overcoming the problem of false negatives due to the hCGβcf, it is possible to perform pregnancy diagnosis more accurately and conveniently by the naked eye.

Furthermore, the concentration measurement range of hCG or hCGβcf is improved by using a chromatography strip for diagnosing pregnancy, which is composed of multiple test lines and control lines.

[Description of Drawings]

FIG. 1 is a schematic diagram showing a cross-section of an immunochromatography strip used in a conventional pregnancy diagnostic kit.
FIG. 2 is a schematic diagram showing an immunochromatography strip for diagnosing pregnancy with multiple test lines used in the pregnancy diagnostic kit of the present invention.

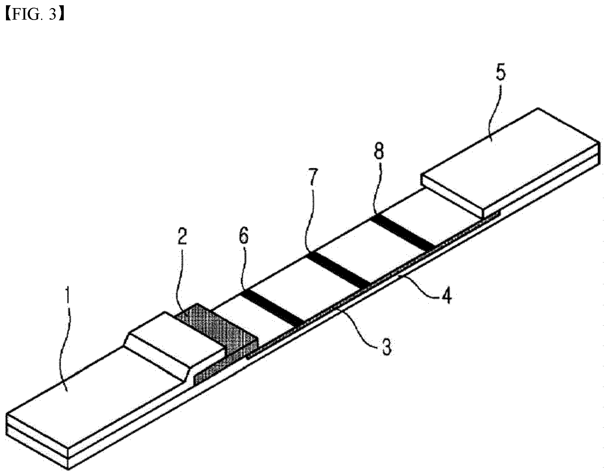

FIG. 3 is a mimetic diagram of an immunochromatography strip for diagnosing pregnancy according to an exemplary embodiment of the present invention.
FIG. 4 is a result of analyzing hCG standard solutions at various concentrations using the pregnancy diagnostic kit of the present invention.
FIG. 5 is a result of analyzing sample solutions in which hCG and hCGβcf are present at various concentrations using the pregnancy diagnostic kit of the present invention.

[Modes of the Invention]

Hereinafter, the present invention will be described in more detail through exemplary embodiments. These exemplary embodiments are intended to illustrate the present invention more specifically.

**[Example 1]**

**Manufacture of immunochromatography strips**

1-1. Manufacture of detection pad formed with test line, heterogeneous test line, and control line

Three analysis lines were dispensed on a nitrocellulose membrane. The nitrocellulose film was laminated on a plastic card using a laminator. Afterwards, as a second anti-human chorionic gonadotropin antibody of test line 1, a monoclonal anti-human chorionic gonadotropin antibody (monoclonal anti-hCG 5014, Medix, Finland) was used, and human chorionic gonadotropin (hCG) was used for test line 2, and an anti-mouse immunoglobulin antibody from goat (anti-mouse antibody from goat, Arista, USA) was used as a ligand for the control line. Each of these was dispensed using an automatic dispenser and then dried at 25°C to 30°C for 2 days (48 hours).

1-2. Manufacture of conjugate pad

A conjugate pad was pre-treated by soaking a pad sufficiently with Tris buffer (10 mM, pH 8.5) containing 0.5% polyvinyl alcohol (PVA) and thoroughly drying in a dryer.
A first conjugate solution, in which colloidal gold particles with a diameter of about 40 nm were combined with a complex clone anti-human chorionic gonadotropin antibody (monoclonal anti-hCG 5006, Medix, Finland) as a first anti-chorionic gonadotropin antibody, was prepared, and a second conjugate solution, in which colloidal gold particles with a diameter of about 40 nm were combined with mouse immunoglobulin (mouse IgG, Arista, USA), was prepared. The first conjugate solution and the second conjugate solution were dispensed on a pre-treated conjugate pad, and after drying completely, it was prepared by cutting it to an appropriate size.

1-3. Manufacture of sample pad

A sample pad was sufficiently soaked in a 0.08 M borate buffer solution containing 1% Triton X-100, 0.5% NaN$_3$, and 0.1% BSA, and after drying completely in a dryer, it was prepared by cutting it into an appropriate size.

1-4. Manufacture of absorbance pad

The absorbance pad was used as it was without any treatment in a state where moisture was completely dried in a dryer, and it was prepared by cutting it into an appropriate size.

1-5. Manufacture of immunochromatography strip

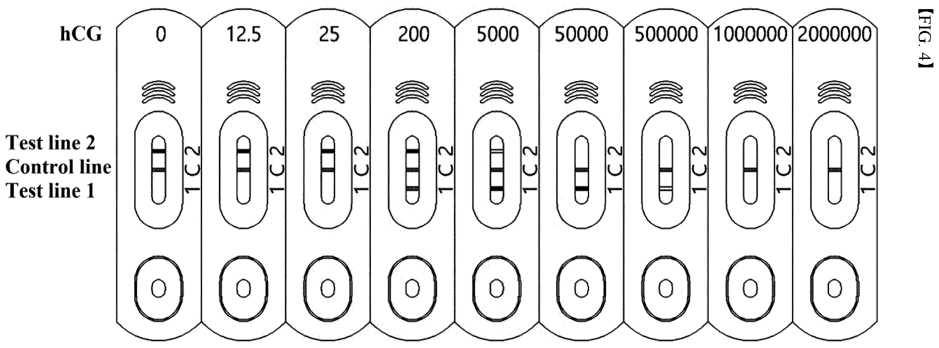

The detection pad, the conjugate pad, the sample pad, and the absorbance pad prepared through each of the above processes were assembled as shown in FIG. 4.
That is, the sample pad was attached so as to overlap with one end of the conjugate pad, one end of the detection pad was attached so as to overlap with the other end of the conjugate pad, and the other end of the detection pad and one end of the absorbance pad were attached to overlap each other. It was cut to about 4 ± 2.0 mm using a cutter to prepare a strip as shown in FIG. 4.
In FIG. 4, the meanings of each reference numeral are as follows.

1: Sample pad; 16 ± 4 × 4 ± 2 mm
2: Conjugate pad of first anti-hCG antibody and gold particles;

$$6 \pm 1.0 \times 4 \pm 2 \text{ mm}$$

3: Nitrocellulose detection pad; 25 ± 5 × 4 ± 2 mm
4: Plastic solid support
5: Absorbance pad; 18 ± 4 × 4 ± 2 mm
6: Test line 1 with immobilized second anti-hCG antibody
7: Control line with immobilized anti-mouse immunoglobulin
8: Test line 2 with immobilized hCG

1-6. Assembly of case

After inserting the manufactured immunochromatography strip for diagnosing pregnancy into the fixed position of a strip in a plastic lower case, the upper case with a sample input port and a result confirmation window was inserted, and then assembled by pressing with a press.

**[Experimental Example 1]**

**Evaluation of chromatography strip for diagnosing pregnancy using hCG standard solutions**

Human hCG standard materials were spiked in non-pregnant urine to prepare standard samples of chorionic gonadotropin and subjected to quantitative and qualitative tests. The prepared standard sample solutions were dispensed by 100 $\mu$L into the sample input port of the case and then developed for 5 minutes. The sample solutions were prepared to have normal human chorionic gonadotropin concentrations at 0, 12.5, 25, 200, 5,000, 50,000, 500,000, 1,000,000, and 2,000,000 mIU/mL, respectively.

FIG. 4 is a diagram showing the development result of the immunochromatography strip for diagnosing pregnancy according to the concentration of normal human chorionic gonadotropin (hCG). Furthermore, the intensity of signals appearing on the control line, test line 1, and test line 2 was confirmed by the naked eye, the intensity of the control line was set to ++, and the generation intensity of generation after the reaction was shown in [Table 1] below.

[Table 1]

| Sample number | 1 | 2 | 3 | 4 | 5 | 6 | 7 | 8 | 9 |
|---|---|---|---|---|---|---|---|---|---|
| Normal hCG concentratio n (mIU/mL) | 0 | 12.5 | 25 | 200 | 5,000 | 50,0 00 | 500, 000 | 1,000, 000 | 2,000, 000 |
| Test line 1 intensity | - | +/- | + | ++ | +++ | +++ | ++ | + | +/- |
| Test line 2 intensity | +++ | +++ | +++ | +++ | + | +/- | - | - | - |
| Control line intensity | ++ | ++ | ++ | ++ | ++ | ++ | ++ | ++ | ++ |

As shown in [Table 1] and [FIG. 4], when only normal hCG was present, a positive response was observed clearly at 25 mIU/mL or more, and at 200 mIU/mL, it showed a reaction of high intensity of ++.

**[Experimental Example 2]**

**Evaluation of chromatography strip for diagnosing pregnancy using sample solutions with hCG and hCGcf**

Next, in order to confirm the accuracy of the analysis method of the present invention when pregnancy was diagnosed with urine including a $\beta$-core fragment, sample solutions were prepared that were spiked with a $\beta$-core fragment and a normal hCG standard material in non-pregnant urine, and these were developed for 5 minutes after dispensing by 100 $\mu$L. The sample solutions were prepared as shown in [Table 2] below.

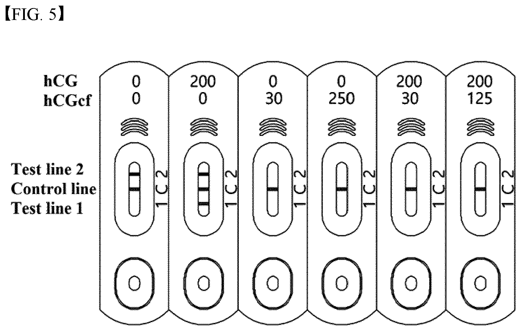

FIG. 5 is a diagram showing the comparison of analysis results depending on the concentrations in samples in which both normal hCG and hCGcf were present. Furthermore, the intensity of signals appearing on the control line, test line 1, and test line 2 was confirmed by the naked eye, the intensity of the control line was set to ++, and the generation intensity of the test line after the reaction was shown in [Table 2] below.

[Table 2]

| Sample | 1 | 2 | 3 | 4 | 5 | 6 |
|---|---|---|---|---|---|---|
| Normal hCG concentration (mIU/mL) | 0 | 200 | 0 | 0 | 200 | 200 |
| $\beta$-core fragment (pmol/mL) | 0 | 0 | 30 | 250 | 30 | 125 |
| Test line 1 intensity | - | ++ | - | - | +/- | - |
| Test line 2 intensity | +++ | +++ | +/- | - | +/- | - |
| Control line intensity | ++ | ++ | ++ | ++ | ++ | ++ |

As shown in [Table 2] and [FIG. 5], in sample 2 in which only hCG was present, it showed a positive response clearly on test line 1 as in Experimental Example 1, but in samples 5 and 6 in which hCGcf was present, even when hCG was at 200 mIU/mL, almost no color was developed on test line 1. From this, it can be confirmed that in the conventional pregnancy diagnosis kit in which only one test line is present, when hCGcf is present, there may be a possibility of false negatives. Since the pregnancy diagnostic kit of the present invention is additionally provided with test lines, pregnancy may be diagnosed by referring to the result of test line 2, when hCGcf is present. In addition, when not only test line 1 shows color development, but also when test line 2 is not visible or is shown at an intensity of the control line or below, it is determined as positive, and thus, it is possible to diagnose pregnancy more accurately.

**Claims**

1. A chromatography strip for diagnosing pregnancy, comprising:

   a conjugate pad; and
   a detection pad,
   wherein the conjugate pad comprises a first anti-human chorionic gonadotropin (hCG) monoclonal antibody and a signal detection labeling material, and
   wherein the detection pad is provided with test line 1 and test line 2 that are isolated, the test line 1 comprises a second-hCG monoclonal antibody, and the test line 2 comprises human chorionic gonadotropin (hCG), wherein:

      (a) the first anti-hCG monoclonal antibody recognizes a β-core fragment site of human chorionic gonadotropin (hCG), and
      wherein the second anti-hCG monoclonal antibody recognizes a different site of hCG which is not the β-core fragment site of hCG;
      (b) the first anti-hCG monoclonal antibody is linked to the signal detection labeling material before or during the development of a sample to form a first conjugate,

         wherein a complex in which hCG or hCG β core fragment (hCGβcf) is bound to the first conjugate is formed when hCG or hCGβcf is present in the sample, and
         wherein as the concentration of hCG or hCGβcf increases in the sample, the number of the formed complexes increases, and conversely, the number of bare conjugates which are not bound to hCG in the sample decreases; and

      (c) a complex in which normal hCG is bound to the first conjugate is captured on test line 1 to indicate a signal, and a complex in which hCGβcf is bound to the first conjugate is not captured on test line 1, and wherein a bare conjugate which is not bound to hCG or hCGβcf in the sample reacts with hCG immobilized at a certain amount on test line 2, thereby being captured on test line 2 to indicate a signal.

2. The chromatography strip of claim 1, wherein the labeling material is colloidal gold, a latex particle, a colored polystyrene microparticle, an enzyme, a fluorescent dye, a conductive polymer, a luminescent material, or a magnetic particle.

3. The chromatography strip of claim 1, wherein the detection pad further comprises a control line for confirming the development of a sample.

4. A method for diagnosing pregnancy as a method for diagnosing pregnancy using the chromatography strip for diagnosing pregnancy according to any one of claims 1 to 3, comprising:

   injecting a sample into the conjugate pad or a pad positioned before to develop; and
   confirming the presence or absence of a signal of a labeling material from test line 1, test line 2, and a control line.

5. The method of claim 4, wherein the control line and test line 2 appear in the case of non-pregnancy, and in the case of pregnancy, test line 1, test line 2, and the control line all appear at a low concentration of hCG, and test line 1 and the control line appear, or only the control line appears at a high concentration of hCG.

6. A pregnancy diagnosis kit as a pregnancy diagnosis kit in which the chromatography strip for diagnosing pregnancy according to any one of claims 1 to 3 is additionally fixed in a case, wherein a guide and a strip support are provided in a lower case, and a sample input port is provided in an upper case; and a result confirmation window is provided at a position corresponding to the test line or the control line.

**Patentansprüche**

1. Chromatographiestreifen zum Diagnostizieren einer Schwangerschaft, umfassend:

   ein Konjugatkissen; und
   ein Nachweiskissen,
   wobei das Konjugatkissen einen ersten monoklonalen Anti-Human-Chorion-Gonadotropin (hCG-) Antikörper und ein Signalnachweismarkierungsmaterial umfasst, und
   wobei das Nachweiskissen mit einer Testlinie 1 und einer Testlinie 2 versehen ist, die isoliert sind, wobei die Testlinie 1 einen zweiten monoklonalen hCG-Antikörper umfasst und die Testlinie 2 humanes Chorion-Gonadotropin (hCG) umfasst, wobei:

      (a) der erste monoklonale Anti-hCG-Antikörper eine β-Kernfragmentstelle von humanem Chorion-Gonadotropin (hCG) erkennt, und wobei der zweite monoklonale Anti-hCG-Antikörper eine andere Stelle von hCG erkennt, die nicht die β-Kernfragmentstelle von hCG ist;
      (b) der erste monoklonale Anti-hCG-Antikörper vor oder während der Entwicklung einer Probe mit dem Signalnachweismarkierungsmaterial verknüpft wird, um ein erstes Konjugat auszubilden,

         wobei ein Komplex, in dem hCG oder hCG-β-Kernfragment (hCGβcf) an das erste Konjugat gebunden ist, ausgebildet wird, wenn hCG oder hCGβcf in der Probe vorhanden ist, und
         wobei mit zunehmender Konzentration von hCG oder hCGβcf in der Probe die Anzahl der ausgebildeten Komplexe zunimmt und umgekehrt die Anzahl der bloßen Konjugate, die nicht an hCG in der Probe gebunden sind, abnimmt; und

      (c) ein Komplex, in dem normales hCG an das erste Konjugat gebunden ist, auf Testlinie 1 eingefangen wird, um ein Signal anzuzeigen, und ein Komplex, in dem hCGβcf an das erste Konjugat gebunden ist, nicht auf der Testlinie 1 eingefangen wird, und
      wobei ein bloßes Konjugat, das nicht an hCG oder hCGβcf in der Probe gebunden ist, mit einem hCG reagiert, das bei einer bestimmten Menge auf Testlinie 2 immobilisiert wird, wobei es dadurch auf Testlinie 2 eingefangen wird, um ein Signal anzuzeigen.

2. Chromatographiestreifen nach Anspruch 1, wobei das Markierungsmaterial kolloidales Gold, ein Latexteilchen, ein gefärbtes Polystyrolmikroteilchen, ein Enzym, ein Fluoreszenzfarbstoff, ein leitfähiges Polymer, ein lumineszierendes Material oder ein magnetisches Teilchen ist.

3. Chromatographiestreifen nach Anspruch 1, wobei das Nachweiskissen ferner eine Kontrolllinie zum Bestätigen der Entwicklung einer Probe umfasst.

4. Verfahren zum Diagnostizieren einer Schwangerschaft als ein Verfahren zum Diagnostizieren einer Schwangerschaft unter Verwendung des Chromatographiestreifens zum Diagnostizieren einer Schwangerschaft nach einem der Ansprüche 1 bis 3, umfassend:

   Injizieren einer Probe in das Konjugatkissen oder ein zuvor positioniertes Kissen für die Entwicklung; und
   Bestätigen des Vorhandenseins oder Nichtvorhandenseins eines Signals eines Markierungsmaterials von Testlinie 1, Testlinie 2 und einer Kontrolllinie.

5. Verfahren nach Anspruch 4, wobei die Kontrolllinie und die Testlinie 2 im Falle einer Nichtschwangerschaft erscheinen und im Falle einer Schwangerschaft die Testlinie 1, die Testlinie 2 und die Kontrolllinie alle bei einer niedrigen Konzentration von hCG erscheinen und die Testlinie 1 und die Kontrolllinie oder nur die Kontrolllinie bei einer hohen Konzentration von hCG erscheinen.

6. Schwangerschaftsdiagnosekit als ein Schwangerschaftsdiagnosekit, bei dem der Chromatographiestreifen zum Diagnostizieren einer Schwangerschaft nach einem der Ansprüche 1 bis 3 zusätzlich in einem Gehäuse fixiert ist, wobei in einem unteren Gehäuse eine Führung und ein Streifenträger bereitgestellt sind und in einem oberen Gehäuse eine Probeneingabeöffnung bereitgestellt ist; und ein Ergebnisbestätigungsfenster an einer Position bereitgestellt ist, die der Testlinie oder der Kontrolllinie entspricht.

**Revendications**

1.  Bandelette de chromatographie pour diagnostiquer une grossesse, comprenant :

    un tampon conjugué ; et
    un tampon de détection,
    dans laquelle le tampon conjugué comprend un premier anticorps monoclonal antigonadotrophine chorionique humaine (hCG) et un matériau de marquage de détection de signal, et
    dans laquelle le tampon de détection est pourvu d'une ligne de test 1 et d'une ligne de test 2 qui sont isolées, la ligne de test 1 comprend un deuxième anticorps monoclonal hCG, et la ligne de test 2 comprend de la gonadotrophine chorionique humaine (hCG), dans laquelle :

    (a) le premier anticorps monoclonal anti-hCG reconnaît un site de fragment central β de la gonadotrophine chorionique humaine (hCG), et
    dans laquelle le deuxième anticorps monoclonal anti-hCG reconnaît un site différent de hCG qui n'est pas le site du fragment central β de hCG ;
    (b) le premier anticorps monoclonal anti-hCG est lié au matériau de marquage de détection de signal avant ou pendant le développement d'un échantillon pour former un premier conjugué,

    dans laquelle un complexe dans lequel hCG ou un fragment central de hCGβ (hCGβcf) est lié au premier conjugué est formé lorsque hCG ou hCGβfcf est présent dans l'échantillon, et
    dans laquelle, à mesure que la concentration de hCG ou de hCGβfcf augmente dans l'échantillon, le nombre de complexes formés augmente et, inversement, le nombre de conjugués nus qui ne sont pas liés à l'hCG dans l'échantillon diminue ; et

    (c) un complexe dans lequel hCG normal est lié au premier conjugué est capturé sur la ligne de test 1 pour indiquer un signal, et un complexe dans lequel hCGβfcf est lié au premier conjugué n'est pas capturé sur la ligne de test 1, et
    dans lequel un conjugué nu qui n'est pas lié à l'hCG ou à l'hCGβfcf dans l'échantillon réagit avec l'hCG immobilisé en une certaine quantité sur la ligne de test 2, étant ainsi capturé sur la ligne de test 2 pour indiquer un signal.

2.  Bande de chromatographie selon la revendication 1, dans laquelle le matériau de marquage est de l'or colloïdal, une particule de latex, une microparticule de polystyrène colorée, une enzyme, un colorant fluorescent, un polymère conducteur, un matériau luminescent ou une particule magnétique.

3.  Bandelette de chromatographie selon la revendication 1, dans laquelle le tampon de détection comprend en outre une ligne de contrôle pour confirmer le développement d'un échantillon.

4.  Procédé pour diagnostiquer une grossesse en tant que procédé pour diagnostiquer une grossesse utilisant la bandelette de chromatographie pour diagnostiquer une grossesse selon l'une quelconque des revendications 1 à 3, comprenant :

    l'injection d'un échantillon dans le tampon conjugué ou un tampon positionné avant le développement ; et
    la confirmation de la présence ou de l'absence d'un signal d'un matériau d'étiquetage provenant de la ligne de test 1, de la ligne de test 2 et d'une ligne de contrôle.

5.  Procédé selon la revendication 4, dans lequel la ligne de contrôle et la ligne de test 2 apparaissent en cas de non-grossesse, et dans le cas d'une grossesse, la ligne de test 1, la ligne de test 2 et la ligne de contrôle apparaissent toutes à une faible concentration d'hCG, et la ligne de test 1 et la ligne de contrôle apparaissent, ou seule la ligne de contrôle apparaît à une concentration élevée d' hCG.

6.  Kit de diagnostic de grossesse comme kit de diagnostic de grossesse dans lequel la bandelette de chromatographie pour diagnostiquer une grossesse selon l'une quelconque des revendications 1 à 3 est en outre fixée dans un boîtier, dans lequel un guide et un support de bandelette sont prévus dans un boîtier inférieur, et un port d'entrée d'échantillon est fourni dans un boîtier supérieur ; et une fenêtre de confirmation de résultat est prévue à une position correspondant à la ligne de test ou à la ligne de contrôle.

【FIG. 1】

Conventional analysis strip

Sample pad — Conjugate pad — Porous membrane pad — Binder — Absorbance pad — Plastic support

【FIG. 2】

Immunochromatography strip for diagnosing pregnancy

【FIG. 3】

【FIG. 4】

【FIG. 5】

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2007172963 A
- KR 20160108695
- CN 106053794
- CN 108931654
- CN 102890157

### Non-patent literature cited in the description

- *Journal of the Korean Society of Emergency Medicine,* 2011, vol. 22 (5), 503-507
- *J. Emergency Medicine,* 2013, vol. 44, 155